# EUROPEAN PATENT SPECIFICATION

(11) **EP 1 294 874 B1**
(45) Date of publication and mention of the grant of the patent: **10.05.2006**
(21) Application number: 01946167.2
(22) Date of filing: 08.06.2001
(51) Int. Cl.: C12N 15/12, C07K 14/705, A61K 48/00, A61P 37/00, A61P 37/06, A61P 29/00

(54) **CD154 VARIANTS AND USES THEREOF**
CD154 VARIANTEN UND DEREN VERWENDUNG
VARIANTS CD154 ET LEURS UTILISATIONS

(30) Priority: 09.06.2000 US 210657 P
(43) Date of publication of application: 26.03.2003
(73) Proprietor: Biogen Idec MA, Inc., Cambridge Massachusetts 02142 (US)
(72) Inventor: HSU, Yen-Ming, Lexington, MA 02420 (US); GARBER, Ellen, Cambridge, MA 02138-1352 (US)
(74) Representative: Vossius & Partner
(86) International application number: PCT/US2001/018517
(87) International publication number: WO 2001/096397

(56) References cited:
- SEYAMA KUNIAKI ET AL: "CD40 ligand mutants responsible for X-linked hyper-IgM syndrome associate with wild type CD40 ligand." JOURNAL OF BIOLOGICAL CHEMISTRY, vol. 274, no. 16, 16 April 1999 (1999-04-16), pages 11310-11320, XP002189389 ISSN: 0021-9258
- GARBER ELLEN ET AL: "CD154 variants associated with hyper-IgM syndrome can form oligomers and trigger CD40-mediated signals." JOURNAL OF BIOLOGICAL CHEMISTRY, vol. 274, no. 47, 19 November 1999 (1999-11-19), pages 33545-33550, XP002189390 ISSN: 0021-9258 cited in the application
- DATABASE BIOSIS [Online] BIOSCIENCES INFORMATION SERVICE, PHILADELPHIA, PA, US; 1996 STUBER ECKHARD ET AL: "Blocking the CD40L-CD40 interaction in vivo specifically prevents the priming of T helper 1 cells through the inhibition of interleukin 12 secretion." Database accession no. PREV199698728286 XP002189392 & JOURNAL OF EXPERIMENTAL MEDICINE, vol. 183, no. 2, 1996, pages 693-698, ISSN: 0022-1007
- SU LIHE ET AL: "Accelerated publication: CD154 variant lacking tumor necrosis factor homologous domain inhibits cell surface expression of wild-type protein." JOURNAL OF BIOLOGICAL CHEMISTRY, vol. 276, no. 3, 19 January 2001 (2001-01-19), pages 1673-1676, XP002189391 ISSN: 0021-9258

## Description

### TECHNICAL FIELD OF THE INVENTION

This invention relates to in vitro methods of using CD154 variants lacking at least a portion of a tumor necrosis factor homologous domain to inhibit the expression of wildtype CD154 on the surface of target cells. The compounds of the invention are useful in treating or inhibiting CD154-dependent immune disorders.

### BACKGROUND OF THE INVENTION

CD154 (i.e., CD40 ligand or CD40L) is a type II membrane protein expressed primarily on activated T cells. The interaction of CD154 with its receptor, CD40, is critical for the functions of T helper cells to induce differentiation, proliferation, and immunoglobulin isotype switching in B cells (for review see Foy et al., *Annu Rev Immunol* 14:591-617 (1996); van Kooten et al., *J Leukoc Biol* 67:2-17 (2000)). The CD154 gene, located at chromosomal region Xq2.6-2.7, spans more than 12 kilobase pairs and contains five exons (Villa et al., *Proc Natl Acad Sci USA* 91:2110-4 (1994)).

The first exon encodes the cytoplasmic region, the transmembrane domain, and six amino acids of the extracellular domain. The second and third exons encode the extracellular stalk region. The fourth and fifth exons encode the C-terminal 147 amino acids (Villa et al., *supra),* a region that shares limited homology with other members of the tumor necrosis factor ("TNF") family and is therefore called the TNF homologous ("TNFH") domain. The X-ray structure of the CD154 TNFH domain reveals that it contains a sandwich-like fold of two β sheets with jellyroll or Greek key topology. Although members of the TNF family share the configuration of the Type II membrane protein, the limited homology between these members is located in the C-terminal TNFH domain of approximately 150 amino acid residues.

Like TNF and lymphotoxin-α, wildtype CD154 exists as trimers (Karpusas et al., *Structure* 3:1031-9 (1995)). The formation of CD154 trimers is mediated by the TNFH domain. It has been shown that the TNFH domain alone is capable of forming trimers (PCT patent application WO 97/00895; Karpusas et al., *supra;* Mazzei et al., *J Biol Chem* 270:7025-8 (1995)). Deletion mutants missing a major portion of this domain do not seem to exist as trimers (Garber et al., *J Biol Chem* 274:33545-50 (1999)). Thus, it appears that the TNFH domain is necessary and sufficient for the assembly of trimeric CD154 proteins.

Mutations of the CD154 gene preventing the expression of functional CD154 protein can lead to an immunodeficiency characterized by elevated IgM levels and low IgG and IgA levels in serum. Since the CD154 gene is located on the X chromosome in humans, this immunodeficiency is called X-linked hyper-IgM syndrome ("XHIM") (Allen et al., *Science* 259:990-3 (1993); Korthauer et al., *Nature* 361:539-41 (1993); DiSanto et al., *Nature* 361:541-3 (1993); Aruffo et al., Cell 72:291-300 (1993); Fuleihan et al., *Proc Natl Acad Sci USA* 90:2170-3 (1993)). Over 70 unique mutations in the CD154 gene have been identified in more than one hundred XHIM patients (Notarangelo et al., *Immunol Today* 17:511-6 (1996)). These mutations are very heterogeneous. They include insertions, deletions, and point mutations. Thus, it is conceivable that the underlying mechanisms for the functional defects of CD154 in XHIM patients are different (Garber et al., *supra*; Seyama et al., *Blood* 92:2421-34 (1998)).

Because the CD154 gene is X-linked, each cell from normal individuals or XHIM patients makes a single species of CD154-encoding transcript. However, in some XHIM patients, mutations in the donor splicing sites (Seyama et al., *supra*; and references cited therein) or the acceptor splicing sites (Ameratunga et al., *Clin Diagn Lab Immunol* 3:722-6 (1996)) lead to generation of multiple species of mRNA transcripts in a single cell. These transcripts include the normally spliced transcripts encoding wildtype CD154, as well as the misspliced transcripts encoding variant CD154 proteins lacking either a major portion of the TNFH domain, or the entire TNFH domain (Seyama et al., *supra;* Ameratunga et al. *supra).*

Because the TNFH domain alone appears to be responsible for the assembly of trimeric CD154 protein, variants lacking the TNFH domain were predicted not to affect the trimerization of the wildtype protein. Thus, it is unclear why patients with mutations at the splicing sites of their CD154 genes exhibit the XHIM syndrome, which presumably results from a lack of functional CD154 protein.

In J. Biol. Chem., vol. 274, 1999, 11310-11320, disclosure is made that CD154 lacking TNFH domain can interact with wild type CD154. Cell surface detection of heterodimers of wild type CD154 and studied mutant CD154 was observed.

### SUMMARY OF THE INVENTION

The present invention is based on our discovery that CD154 variants lacking at least a portion of the TNFH domain can nonetheless interact with a wildtype CD154 protein via the stalk region. This interaction retains the wildtype CD154 protein intracellularly, thereby preventing the wildtype protein from being expressed on the cell surface and participating in any CD154 functional activity. This discovery reveals the stalk region of the CD154 protein as a previously unrecognized structural element that contributes to CD154 trimer assembly.

Accordingly, this invention provides an in vitro method of decreasing (including inhibiting) the expression of wildtype CD154 protein on the surface of target cells. In this in vitro method, a recombinant nucleic acid construct is introduced into a CD154-expressing target cell, where the construct is capable of directing the expression of a CD154 variant protein lacking a functional TNFH domain of the wild type protein, such that the variant protein is incapable of trimerization and yet can bind to the wildtype protein, rendering the wildtype protein unable to reach the cell surface. For instance, the variant lacks at least a portion of the TNFH domain. By way of example, a CD154 variant useful in the invention may lack at least 5 (e.g., at least 10; at least 15; or at least 20) amino acids of the TNFH domain. In one embodiment, a CD154 variant may lack the entire TNFH domain. In another embodiment, a CD154 variant may comprise a TNFH domain with insertional mutations and/or point mutations.

As used herein, the TNFH domain of CD154 corresponds to a region spanning from amino acid residues 116 to 261 of SEQ ID NO:1:

SEQ ID N0:1 represents a full-length sequence of human CD154, and is available in GenBank under accession number CAA48554 (referencing Graf et al., *Eur. J*. *Immunol.* 22(12): 3191-4 (1992)). Allelic isoforms of SEQ ID NO:1 can also be used as the cognate sequence for the CD154 variants useful in this invention.

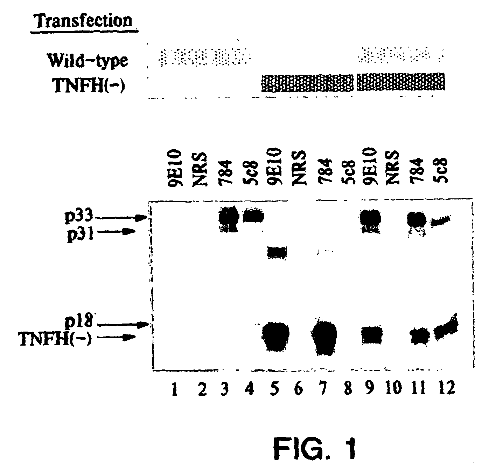

Examples of CD154 variants useful in this invention include those that lack (1) amino acid residues 116-136, (2) amino acid residues 137-261, (3) amino acid residues 115-261, and (4) amino acid residues 97-261, respectively, of SEQ ID NO:1. Methods of generating CD154 variants are known in the art. See, e.g., Fig. 1 and Table 1 of Garber et al., *J. Biol. Chem.* 274:33545-550 (1999).

This invention further provides compounds for use in a method of treating or inhibiting a CD154-dependent disease (e.g., an immune disorder mediated by CD154:CD40 interaction) in a subject (i.e., a mammal such as a primate, preferably a human). The method involves administering to target cells of the subject, such as T cells (e.g., CD4⁺ and CD8⁺ T cells) or megakaryocytes, a CD154 variant of the present invention, thereby decreasing the expression of wildtype CD154 on the surface of the target cells. In one embodiment, the CD154 variant is generated inside the target cell, based on uptake by the cell of a gene expression construct comprising a nucleic acid sequence encoding that variant.

Within the scope of the present invention are also pharmaceutical compositions comprising a nucleic acid construct that directs expression of a CD154 mutant useful in a method of the invention. The invention also provides the use of such a construct for the manufacture of a medicament for decreasing the expression of wildtype CD154 on the surface of a target cell, and/or for treating a patient suffering from or predisposed to a CD154-mediated disease.

The present invention is well-suited for local delivery and uptake by target cells at a locus in need of therapeutic treatment in the body of the subject. For example, the gene expression construct described herein can be administered locally (e.g., by injection) to spleen or other lymphoid tissues, or into a locus of inflammation, such as a wound site or pathological lesion. Such local treatment circumvents any complications that may be encountered with systemic delivery of immunomodulating agents.

In addition, the present gene expression construct avoids potential drawbacks of extracellular immunomodulating agents, such as antibodies. Antibodies, when bound to a cell-surface antigen, may trigger cell destruction, for instance, via an antibody-dependent cell cytotoxicity response, or by macrophages or other effector cells that display Fc receptors on their cell surface. Such cell destruction may not be desirable in certain therapeutic settings.

Other features and advantages of the present invention will be apparent from the following drawings and detailed description, and also from the appended claims.

Unless otherwise defined, all technical and scientific terms used herein have the same meaning as commonly understood by one of ordinary skill in the art to which this invention pertains. Exemplary methods and materials are described below.

### BRIEF DESCRIPTION OF THE DRAWINGS

Fig. 1 is an autoradiograph demonstrating the association of TNFH(-) CD154 with wildtype CD154.
Fig. 2 is a panel of autoradiographs demonstrating the dose-dependent inhibitory effect of TNFH(-) CD154 on the production of functional CD154 trimers.
Figs. 3A and 3B are a bar graph and a table, respectively, showing that a CD154 mutant reduces the lymphotoxin-α up-regulating activity of wildtype CD154.
Fig. 4 is a panel of autoradiographs demonstrating that TNFH(-) CD154 prevents the cell surface expression of wildtype CD154.
Fig. 5 is a schematic diagram illustrating that TNFH(-) CD154 binds to wildtype CD154 intracellularly and prevents it from reaching the cell surface.

### DETAILED DESCRIPTION OF THE INVENTION

It has been established in the past decades that T cell activation requires both T cell antigen receptor ("TCR") mediated signals and simultaneously delivered costimulatory signals. For example, antibody production by B cells in response to protein antigens requires an antigen-specific interaction between B cells and helper T cells as well as non-antigen-specific, costimulatory receptor-ligand interactions between the B and T cells. These non-antigen-specific interactions include the binding of CD40 on B cells to CD154 on T cells.

Human CD40 is a 50 kD cell surface protein expressed on mature B cells, macrophages and activated endothelial cells. CD40 belongs to a class of receptors involved in proliferation and apoptosis, including Fas/CD95, TNF receptors and lymphotoxin receptors. Human CD154 is a 32 kD type II membrane glycoprotein transiently expressed primarily on activated T cells. CD40:CD154 interaction is required for essentially all T cell-dependent immune responses, including antibody responses. In particular, CD40:CD154 interaction provides anti-apoptotic and/or lymphokine stimulatory signals.

The present invention rests on the surprising discovery that CD154 mutational variants lacking at least a portion of the TNFH domain can bind to wildtype CD154 intracellularly and prevent the wildtype protein from reaching the cell surface. Thus, expression of such a CD154 variant in the T cells of a subject can specifically suppress CD154-dependent immune responses by eliminating the cell surface expression of wildtype CD154.

### Treatment of Diseases

CD40:CD154 interaction is known to be pivotal in the induction of T cell dependent immune responses, including antibody-mediated humoral immune responses and T-cell mediated inflammatory responses to protein antigens. Although considerable efforts have been invested by several groups of investigators in developing means for intervening in this interaction to avert or to treat unwanted or pathological immune responses, there remains a need for improved means of interrupting CD40:CD154 interaction in therapeutic settings.

The present invention provides the improved means. According to this invention, a CD154 variant lacking a functional TNFH domain (e.g., a TNFH-minus CD154 mutant) can be introduced into target T cells in a subject to treat (e.g., mitigate, delay or reverse) or inhibit (e.g., prevent the onset or progression of) CD154-dependent diseases that may be characterized by significant inflammatory system or immune system involvement. Such diseases include, but are not limited to, lupus, systemic lupus erythematosis, lupus nephritis, lupus neuritis, asthma, chronic obstructive pulmonary disease, bronchitis, emphysema, multiple sclerosis, uveitis, Alzheimer's disease, traumatic brain injury, traumatic spinal cord injury, stroke, atherosclerosis, coronary restenosis, ischemic congestive heart failure, cirrhosis, hepatitis C, diabetic nephropathy, glomerulonephritis, autoimmune disease, osteoarthritis, rheumatoid arthritis, psoriasis, atopic dermatitis, systemic sclerosis, radiation-induced fibrosis, Crohn's disease, ulcerative colitis, multiple myeloma, ocular inflammatory disease, graft versus host disease, graft rejection (e.g., corneal and retinal graft rejection) or cachexia.

The CD154 mutational variants can also be administered prophylactically to a patient who has not yet shown symptoms of the diseases. For instance, the CD154 variants can be used to treat patients who will undergo transplantation so as to prevent or mitigate possible graft rejection.

In one embodiment, the CD154 variant protein can be introduced to a target cell by local injection of liposomes or other suitable carriers (e.g., microspheres) that contain the variant protein. For enhanced targeting, the liposomes or other suitable carriers may be coated with molecules which function as ligands of tissue-specific receptors.

### Gene Therapy

According to this invention, a TNFH-minus CD154 mutant may also be introduced into a target cell by expressing within the cell a nucleic acid construct comprising a promoter sequence operably linked to a sequence encoding the mutant CD154 protein.

### (1) VECTORS

A nucleic acid construct according to this invention may be derived from a non-replicating linear or circular DNA or RNA vector, or from an autonomously replicating plasmid or viral vector. Alternatively, the construct may be integrated into the host genome. Any vector that can transfect or transduce a T cell may be used. Preferred vectors are viral vectors, including those derived from replication-defective retroviruses (see, e.g., WO 89/07136; Rosenberg et al., *N*. *Eng, J. Med*. 323(9): 570-578 (1990)), adenovirus (see, e.g., Morsey et al., *J. Cell. Biochem.,* Supp. 17E (1993)), adeno-associated virus (Kotin et al., *Proc. Natl. Acad. Sci. USA* 87:2211-2215 (1990)), replication-defective herpes simplex viruses (HSV; Lu et al., Abstract, page 66, Abstracts of the Meeting on Gene Therapy, Sept. 22-26, 1992, Cold Spring Harbor Laboratory, Cold Spring Harbor, New York), vaccinia virus (Mukherjee et al., *Cancer Gene Ther.* 7:663-70 (2000)), and any modified versions of these vectors. Methods for constructing expression vectors are well known in the art. *See, e.g.,* Sambrook et al., *Molecular Cloning: A Laboratory Manual,* Cold Spring Harbor Laboratory, 2nd Edition, Cold Spring Harbor, New York, 1989).

The vectors of this invention may target T cells specifically. T-cell-specific viral vectors useful in gene therapy are known in the art. For instance, one can use (1) the retroviral vectors of Annenkov et al., *Gene Therapy* 7:714-22 (2000); Cavazzana-Calvo et al., Science 288:669-72 (2000); and Farson et al., *J. Gene Med.* 1:195-209 (1999); (2) the herpesvirus saimiri vector of Hiller et al., *Gene Therapy* 7:664-74 (2000); (3) the HIV-based hybrid vectors of Kung et al., *J*. *Virol.* 74:3668-81 (2000); (4) the HIV-derived lentiviral vectors of Costello et al., *Gene Therapy* 7:596-604 (2000) ; or (5) any modified versions of the above-mentioned vectors.

### (2) EXPRESSION CONTROL SEQUENCES

In these vectors, expression control sequences are operably linked to the nucleic acid sequence encoding the mutant protein of the invention. Any expression control sequences than can direct a desired level of transcription in T cells may be used. For eukaryotic cells, expression control sequences may include a promoter, an enhancer, such as one derived from an immunoglobulin gene, SV40, cytomegalovirus, etc., and a polyadenylation sequence. A nucleic acid construct of this invention may also contain an internal ribosome entry site ("IRES"), and an intron that may be desirably located between the promoter/enhancer sequence and the mutant CD154-coding sequence. Selection of these and other common vector elements are conventional. See, e.g., Sambrook et al, *supra;* Ausubel et al.*, Current Protocols in Molecular* Biology, John Wiley & Sons, New York, (1989); and references cited therein.

In one embodiment of the present invention, the native promoter for CD154 is used. The native promoter may be preferred when it is desired that expression of mutant CD154 should mimic the native expression. The native promoter may be used when expression of mutant CD154 must be regulated temporally or developmentally, or in a tissue-specific manner, or in response to specific native transcriptional stimuli. In a further embodiment, other native expression control elements, such as enhancer elements, polyadenylation sites or Kozak consensus sequences may also be used to mimic the native expression.

In another embodiment of the present invention, T-cell-specific promoters are desired. Such promoters include two classes: cell type specific promoters and activation specific promoters. Examples of such promoters include, without limitation, promoters derived from the genes of CD2, CD4, CD3, T cell receptor α and β chains and IL-2.

To prevent prolonged immunosuppression, it may be desirable to use inducible promoters to regulate the expression of mutant CD154. Such promoters are known in the art. They include, without limitation, (1) tetracycline-inducible promoters (Gossen et al., *Science* 268:1766-1769 (1995); Harvey et al., *Curr. Opin. Chem. Biol.* 2:512-518 (1998)) ; (2) tetracycline-suppressible promoters (Alvarez-Vallina et al., *Cancer Gene Therapy* 7:526-9 (2000); Gossen et al, *Proc. Natl. Acad. Sci. USA* 89:5547-5551 (1992)); (3) the rapamycin-inducible promoter systems of Ye et al., *Science* 283:88-91 (1999); Magari et al., *J. Clin. Invest.* 100:2865-2872 (1997); and Rivera et al., *Nat. Medicine* 2:1028-1032 (1996); (4) the zinc-inducible metallothionine promoter; (5) the dexamethasone (Dex)-inducible mouse mammary tumor virus (MMTV) promoter; (6) the T7 polymerase promoter system (WO 98/10088); (7) the ecdysone insect promoter (No et al, *Proc. Natl. Acad. Sci. USA* 93:3346-3351 (1996)); (8) the RU486-inducible promoter systems (Wang et al., *Nat. Biotech.* 15:239-243 (1997); Wang et al., *Gene Ther.* 4:432-441 (1997)); and (9) the modified versions of the the above promoter systems. Other types of inducible promoters useful in this invention are those regulated by a specific physiological state, e.g., temperature, acute phase, or in replicating cells only.

In yet another embodiment of the present invention, high-level constitutive expression is desired. Exemplary promoters for this purpose include, without limitation, the retroviral Rous sarcoma virus (RSV) LTR promoter/enhancer, the cytomegalovirus (CMV) immediate early promoter/enhancer (see, e.g., Boshart et al, *Cell* 41:521-530 (1985)), the SV40 promoter, the dihydrofolate reductase promoter, the cytoplasmic β-actin promoter and the phosphoglycerol kinase (PGK) promoter.

Using the guidance provided by this application, one of skill in the art may make a selection among the above expression control sequences and modified versions thereof without departing from the scope of this invention.

### (3) ADMINISTRATION OF NUCLEIC ACID CONSTRUCTS

The nucleic acid constructs of this invention may be formulated as a pharmaceutical composition for use in any form of transient and/or stable gene transfer *in vivo* and *in vitro.* The composition comprises at least the nucleic acid construct and a pharmaceutically acceptable carrier such as saline. Other aqueous and non-aqueous sterile suspensions known to be pharmaceutically acceptable carriers and well known to those of skill in the art may be employed also. The construct may be used for *in vivo* and ex vivo gene therapy, *in vitro* protein production and diagnostic assays.

The nucleic acid construct can be introduced into target cells as naked DNA, or by, e.g., liposome fusion (see, e.g., Nabel et al., *Science* 249:1285-8 (1990); Ledley, *J Pediatrics* 110:1-8 and 167-74 (1987); Nicolau et al., *Proc Natl Acad Sci USA* 80:1068-72 (1983)), erythrocyte ghosts, or microsphere methods (microparticles; see, e.g., United States patents 4,789,734, 4,925,673, and 3,625,214; Gregoriadis, *Drug Carriers in Biology and Medicine,* pp. 287-341, *Academic* Press, 1979). Alternatively, the nucleic acid construct can be coupled to ligands of T-cell-specific receptors, and thereby enter T cells via receptor-mediated endocytosis.

If the nucleic acid construct is viral-based, it can also be packaged as a virion which then is used to transduce a cell (e.g., an autologous T cell isolated from a patient) *ex vivo.* The infected cell is then returned to the body of the patient. Alternatively, the recombinant virus may be administered to a patient directly, e.g., intravenously, intraperitoneally, intranasally, intramuscularly, subcutaneously, and/or intradermally, as determined by one skilled in the gene therapy art. A slow-release device, such as an implantable pump, may be used to facilitate delivery of the recombinant virus to a cell. Where the virus is administered to a subject, the specific cells to be infected may be targeted by controlling the method of delivery. For example, intravenous injection of the virus may be used to facilitate targeting the virus to a circulating T cell. Target areas of the body for local delivery sites include, for example, the lungs, skin, lymph nodes, thymus, spleen and bone marrow. Such delivery may be, for example, by topical, inhalation, aerosol or local injection routes, including, for example, portal vein catheters. The treatments of the invention may be repeated as needed, as determined by one skilled in the art.

Dosages of the nucleic acid construct of this invention in gene therapy will depend primarily on factors such as the condition being treated. The dosage may also vary depending upon the age, weight and health of the patient. For example, an effective human dosage of a mutant CD154-coding virus is generally in the range of from about 0.5 ml to 50 ml of saline solution containing the virus at concentrations of about 1 x 10⁷, 1 x 10⁸, 1 x 10⁹, 1 x 10¹⁰, 1 x 10¹¹, 1 x 10¹², 1 x 10¹³, 1 x 10¹⁴, 1 x 10¹⁵, or 1 x 10¹⁶ viral particles per dose administered. The dosage will be adjusted to balance the corrective benefits against any adverse side effects. The levels of expression of mutant CD154 may be monitored to determine the type and frequency of dosage administration.

The pharmaceutical compositions of the invention may be used alone or in a mixture, or in chemical combination, with one or more materials, including other proteins or recombinant vectors that increase the biological stability of the proteins or the recombinant vectors, or with materials that increase the compositions' ability to target T cells selectively.

### Combined Therapy

In addition, the pharmaceutical compositions of the invention can be used in combination with another immunomodulating regimen to achieve desired immunosuppression, e.g., long-term, rejection-free integration of heterologous donor tissue into a primate recipient. For instance, an agent that blocks the CD154:CD40 interaction, or blocks costimulation via CD28, CD80 or CD86 can be used.

Exemplary CD154:CD40 interaction inhibitors are antibodies against CD154, such as monoclonal antibodies ("mAbs") 5c8 (produced by the hybridoma having ATCC Accession Number HB 10916; disclosed in United States patent 5, 474, 771) ; ImxM90, ImxM91 and ImxM92 (described in United States patent 5,961,974); and those commercially available from Ancell (clone 24-31, catalog # 353-020, Bayport, MN), Genzyme (Cambridge, MA, catalog # 80-3703-01), and PharMingen (San Diego, catalog #33580D). Numerous additional anti-CD154 antibodies have been produced and characterized (see, e.g., PCT patent application WO 96/23071 of Bristol-Myers Squibb).

Other known immunomodulators that block CD154:CD40 interaction include anti-CD154 molecules of other types, such as complete Fab fragments, F(ab')₂ compounds, V_{H} regions, F_{V} regions, single chain antibodies (see, e.g., PCT patent application WO 96/23071), polypeptides, fusion proteins (such as CD40Ig, as in Hollenbaugh et al., *J*. *Immunol. Meth.* 188:1-7 (1995)), and small molecule compounds such as small semi-peptidic compounds or non-peptidic compounds. All of these immunomodulators are capable of blocking or interrupting CD40:CD154 interaction. Procedures for designing, screening and optimizing small molecules are provided in PCT patent publication WO 97/00895, the specification of which is hereby incorporated by reference.

To avoid potential immune responses to the recombinant virus during gene therapy, it may also be desired to adopt the treatment regimen identical or similar to that described in Chirmule et al., *J*. *Virol.* 74:3345-52 (2000). In this regimen, a patient is first treated concurrently with (1) the recombinant virus without the mutant CD154 insert and (2) an immunomodulator such as a humanized anti-CD154 antibody or another costimulation inhibitor. Then the patient is treated with the mutant CD154-coding virus. It has been demonstrated that such a two-step regimen results in siginificant and prolonged inhibition of the recombinant virus-specific humoral response that often causes side effects in gene therapy patients.

See also United States patent 5, 872, 174 and PCT patent application WO 96/26285.

### Pre-Clinical Model Systems for Evaluating Mutant CD154 Gene Therapy Regimens

An exemplary model system for testing efficacy of mutant CD154 gene therapy regimens is the primate renal allograft model disclosed in Kirk et al., *Proc. Natl. Acad. Sci. USA* 94:8789-94 (1997), the teachings of which are incorporated by reference herein. This rhesus monkey model can serve as a rigorous test of immune manipulation: one that is exquisitely sensitive to even minor changes in allograft function or adverse effects on recipient wound healing and immune system function. In addition, it has biological similarity to human renal transplantation: specifically, genes that encode MHC proteins are well conserved between rhesus monkeys and humans, and rhesus monkeys' rejection of vascularized organs closely parallels that seen clinically.

It will be readily appreciated that this model system is suitable for evaluating grafts comprising renal (kidney) tissue. Other art-recognized preclinical model systems, preferably primate model systems, are suitable for assessing efficacy of mutant CD154 gene therapy in suppressing rejection of other graft tissue types such as liver, heart, lung, pancreas, pancreatic islet, skin, peripheral and central nerve. In addition, efficacy of mutant CD154 gene therapy according to the present invention may be assessed in animal models of lupus nephritis, such as those described in PCT patent applications WO 98/30240 and WO 98/30241. Such efficacy may also be assessed in animal corneal allograft models, such as murine corneal allograft models.

### EXAMPLES

The following examples illustrate the methods and materials of the present invention.

### Example 1: Procedures

### (1) Cell lines, Antibodies, and Ig Fusion Protein

BJAB, a human B cell line, was a gift from Dr. George Mosialos at Harvard Medical School. The cell line was maintained in a RPMI medium supplemented with penicillin, streptomycin, 10% heat inactivated fetal bovine serum, and 4 mM glutamine. The mAb 9E10 was produced and purified from the culture of the 9E10 hybridoma, available from the American Type Culture Collection. See also G.I. Evan et al., "Isolation of Monoclonal Antibodies Specific for Human c-myc Proto-Oncogene Product", *Mol Cell Biol* 5:3610-3616 (1985) regarding mAb 9E10 and the EQKLISEEDL myc tag. Procedures for engineering, producing and purifying CD40-Fc fusion protein, humanized 5c8, and rabbit polyclonal antibodies Rb779 and Rb784 were carried out as described in Hsu et al., *J Biol Chem* 272:911-5 (1997).

### (2) Transient Expression of Wildtype and Mutant CD154 Proteins

A wildtype CD154 cDNA was isolated from a cDNA library made from activated human peripheral blood cells. The coding sequence of this cDNA encodes a protein having an amino acid sequence of SEQ ID NO:1. Procedures for constructing mutants of CD154 were carried out as described in Garber et al., *supra.* The wildtype and mutant cDNAs were subcloned or reconstructed from restriction fragments with confirmed sequence into a unique NotI site in a CMV, immediate early promoter-driven, expression vector containing an SV40 origin for amplification in COS7 cells. COS7 cells were transfected with supercoiled plasmid DNA using lipofectamine (GibcoBRL, Grand Island, New York) following the manufacture's instructions. Plasmid DNA lacking the CD154 coding sequence was used as a negative control, and a vector containing the wild-type CD154 coding sequence was used as a positive control in all examples. Expression of CD154 and its variants was analyzed on transfected cells harvested 72 hours after transfection. Metabolic labeling and biotinylation of cell surface proteins, immunoprecipitation, SDS-polyacrylamide gel electrophoresis ("SDS-PAGE"), and western blotting analysis were performed using procedures described in Hsu et al., *supra.*

Preparation of membrane fractions from transfected COS7 cells and the procedures for analysis of lymphotoxin-α upregulation were performed as previously described (Garber et al., *supra*).

### Example 2: TNFH(-) mutant is associated with wildtype CD154.

To investigate the effect of TNFH(-) mutant on the expression of wildtype CD154, lysates of metabolically labeled COS7 cells co-transfected with cDNAs encoding these proteins were analyzed by immunoprecipitation using CD154-specific antibodies. To distinguish the mutant protein from the p18 component of the wildtype heterotrimeric complexes (Hsu et al., *supra*), a myc tag was engineered at the C-terminal of the first 96 amino acids of CD154 to replace the non-CD154 amino acids (11 or 21 amino acids) predicted in the aberrantly spliced transcripts (patients 19, 20 and 21 in Seyama et al., *supra.*

To determine the association of CD154 mutants with the wildtype protein, cell lysates prepared from ³⁵S-metabolically labeled COS7 cells transfected with cDNA encoding either the full length wildtype or the myc-tagged mutant CD154 (amino acid residues 1-96 of SEQ ID NO:1) or both were immunoprecipitated with anti-myc mAb 9E10, anti-human CD154 mAb 5c8, or anti-CD154-C-terminal peptide antiserum Rb784 (or "784"). Immunoprecipitates were analyzed by electrophoresis on 10-20% gradient SDS-PAGE gel, followed by autoradiography (Fig. 1).

Fig. 1 shows that when wildtype CD154 was expressed alone, immunoprecipitates of anti-CD154-N-terminal peptide antiserum Rb784 (lane 3) and anti-CD154 mAb 5c8 (lane 4) contained primarily the full length p33 protein, some p31 protein, and a small amount of p18 (p33, p31 and p18 are components of wildtype CD154). This is consistent with our previous observation (Hsu et al., *supra) .* The p33, p31 and p18 components were not observed in the immunoprecipitates of either anti-myc mAb 9E10 (lane 1) or the control rabbit antiserum (lane 2). When a mutant CD154 protein (containing amino acid residues 1-96 OF SEQ ID NO:1 and linked to a myc tag) ("TNFH(-)") was expressed alone, a p17 component, corresponding to the mutant protein itself, was immunoprecipitated by both 9E10 and Rb784 but not by mAb 5c8 or the control rabbit antiserum (lanes 5 to 8). When mutant and wildtype proteins were co-expressed, immunoprecipitates of 9E10 contained not only the p17 myc-tagged mutant protein but also the p33, p31, and p18 wildtype proteins (lane 9). Similar protein patterns were found in immunoprecipitates of Rb784 and mAb 5c8, but not the control serum (lanes 10 to 12). Notably, mAb 5c8 immunoprecipitates exhibited a less amount of these proteins, suggesting that not all of the wildtype proteins expressed were recognized by 5c8.

The above data reveal three important findings. First, mutant CD154 proteins can be stably produced and readily detected. Second, mutant CD154 proteins missing the TNFH domain can associate with wildtype CD154. Third, at least some wildtype proteins, while associated with the mutant proteins, can interact with mAb 5c8 whose binding epitope has been shown to be conformational and similar to the CD154 binding site for CD40.

### Example 3: Association of TNFH(-) mutant protein diminishes the receptor-binding activity of the wildtype protein in a dose dependent fashion.

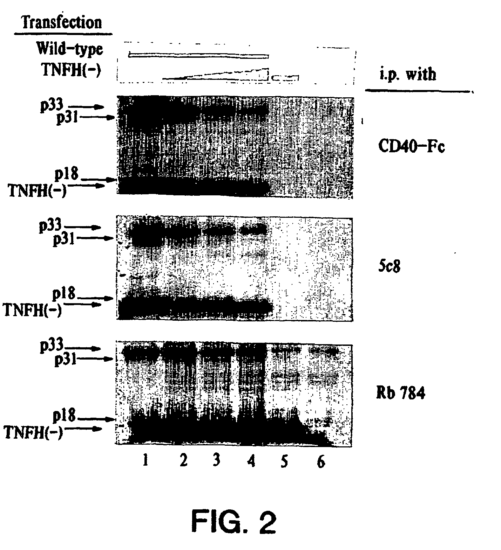

The data shown in Fig. 1 suggest that the association of TNFH(-) CD154 with wildtype CD154 may compromise the ability of the wildtype protein to interact with its receptor. To further examine this, we transfected COS7 cells with (1) a constant amount of a plasmid containing cDNA encoding full length CD154 and a varied amount of a plasmid containing cDNA encoding TNFH(-) CD154. The transfectants were then metabolically labeled with ³⁵S and lysed. Immunoprecipitates of the lysates were analyzed by a 10-20% gradient SDS-polyacrylamide gel followed by autoradiography (Fig. 2).

Fig. 2 shows that the amount of wildtype CD154 immunoprecipitated by Rb784 was the same regardless of the amount of TNFH(-) DNA used for transfection (Fig. 2, bottom panel). In lane 1, the cells were transfected with only the plasmid encoding wildtype CD154. In lanes 2-4, the cells were transfected with the same amount of the wildtype CD154 plasmid and a varied amount of the TNFH(-) plasmid; and the ratios of the former plasmid to the latter plasmid were 3:1, 1:1, and 1:3, respectively. In lane 6, the cells were transfected with only a control plasmid.

The data shown in Fig. 2 indicates that the expression of the wildtype protein was not affected by the introduction of the TNFH(-) mutant. However, the amount of wildtype protein recognized by a CD40-Fc fusion protein or by mAb 5c8 was inversely proportional to the amount of the TNFH(-) plasmid used for transfection (Fig. 2, top and middle panels, respectively). Interestingly, only a small portion of the total TNFH(-) mutant expressed (Fig. 2, bottom panel) was immunoprecipitated by either CD40-Fc or mAb 5c8. These results show that expression of the TNFH(-) mutant affected the receptor-interacting activity, but not the expression level, of wildtype CD154 in a dose dependent fashion.

### Example 4: Effect of TNFH(-) mutant protein on the function of wildtype CD154.

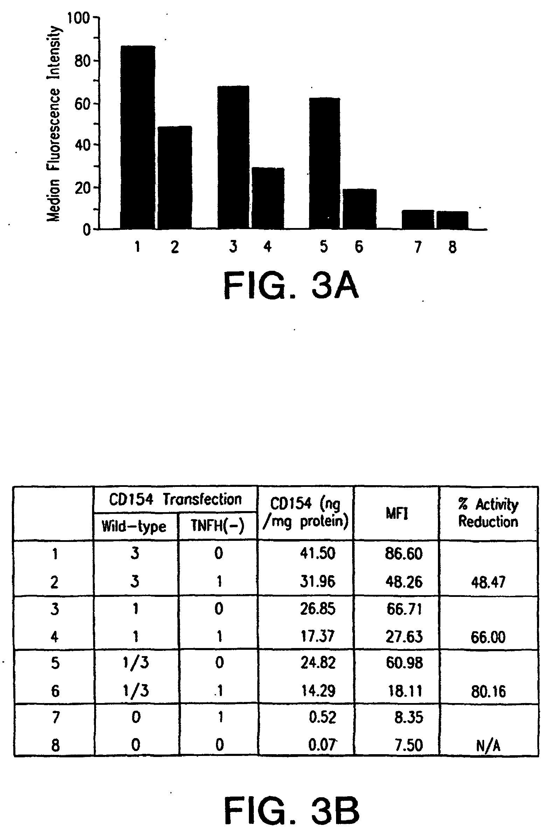

To test if the co-expression of mutant CD154 affects the function of the wildtype protein, UV-irradiated membranes from transfected COS7 cells transfected with cDNA(s) encoding wildtype and/or mutant CD154 were incubated with BJAB cells for 24 h. Ratios of wildtype to mutant cDNAs used for COS7 transfections are indicated in Fig. 3B. The BJAB cells were stained with biotin-labeled anti-lymphotoxin-α mAb, NC2, then with phycoerythrin-labeled streptavidin, and finally fixed with 1% paraformaldehyde. FACS analysis of the BJAB cells was subsequently used to determine the level of up-regulated lymphotoxin-α on the surface of the cells (Fig. 3A, in which the bars represent the mean fluorescence intensities).

Figs. 3A and 3B show that co-expression of the mutant CD154 protein with the wildtype protein reduced the lymphotoxin-α up-regulating ability of the wildtype protein present on the plasma membranes. This inhibitory effect was dose-dependent. When the amount of wildtype cDNA used for transfection was one third of the mutant cDNA, the functional activity of CD154 was down to the background level.

### Example 5: TNFH (-) mutant does not express on the cell surface.

There are at least two possible mechanisms by which mutant CD154 inhibits wildtype CD154 functions in a dose-dependent manner. First, a wildtype CD154 associated with a mutant CD154 may be less active in interacting with CD40. Second, a wildtype CD154 associated with a mutant CD154 may not be functional at all and the activity observed in the up-regulation of lymphotoxin-α may be due exclusively to the wildtype proteins that are free from association with mutant proteins. To distinguish these two possibilities, we next examined the biochemical properties of CD154 proteins expressed on the cell surface.

To do this, COS7 cells were transfected on day one with cDNA encoding wildtype CD154, or cDNA encoding the TNFH(-) mutant, or both. The transfectants were rinsed on day four with PBS to remove cell debris and unattached cells, and then labeled with biotin *in situ*. Labelling was carried out using biotin-sulfo-NHS at 0.5 mg/ml in water left on the cells for 3 minutes at room temperature. The reaction was stopped using glycine, with the transfectant cells remaining in a vast molar excess of glycine for 15 minutes, after which they were rinsed with PBS.

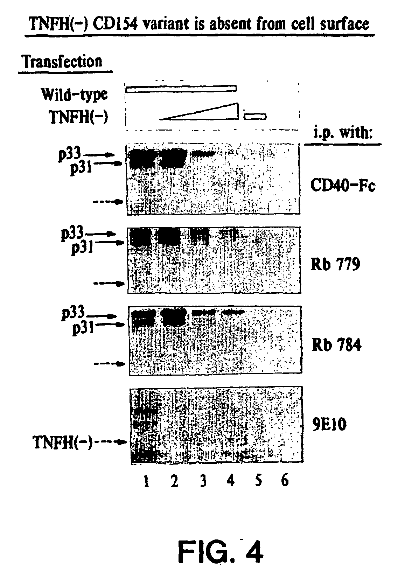

The transfectant cells were then lysed, centrifuged to remove cell debris, and immunoprecipitated with CD40-Fc, Rb779, Rb784, or 9E10. Immunoprecipitates were subjected to electrophoresis in 10-20% SDS-polyacrylamide gel, transferred to nitrocellulose membrane and probed with horseradish peroxidase-conjugated streptavidin (Fig. 4).

Fig. 4 shows that CD40-Fc fusion protein, Rb784, and Rb779, but not 9E10, immunoprecipitated the biotin-labeled wildtype proteins. In addition, the amount of wildtype CD154 detected at the cell surface was inversely proportional to the ratio of mutant cDNA to wildtype cDNA used for co-transfection. Thus, the expression of the TNFH(-) mutant protein prevented the surface expression of the wildtype protein in a dose-dependent fashion. Importantly, we did not detect biotin-labeled mutant protein (p17) in immunoprecipitates with CD40-Fc fusion protein, Rb784, and Rb779, suggesting that the biotin-labeled, i.e. cell surface expressed, wildtype proteins were not associated with the TNFH(-) mutant protein. Since there is a total of eight lysine residues in the extracellular domain of the TNFH(-) mutant, it was unlikely that none of these lysine residues was accessible for biotinylation under the experimental conditions such that mutant proteins present on the surface were undetected. Moreover, the fact that 9E10 did not immunoprecipitate any wildtype proteins indicates that none of the cell surface wildtype protein was associated with the TNFH(-) mutant.

Together with the results shown in Fig. 1, these data indicate that wildtype CD154 proteins free of association with the TNFH(-) mutant were expressed on the cell surface. However, wildtype proteins complexed with the TNFH(-) mutant were retained inside the cell. Thus, association with the TNFH(-) mutant prevents wildtype CD154 from being expressed on the cell surface.

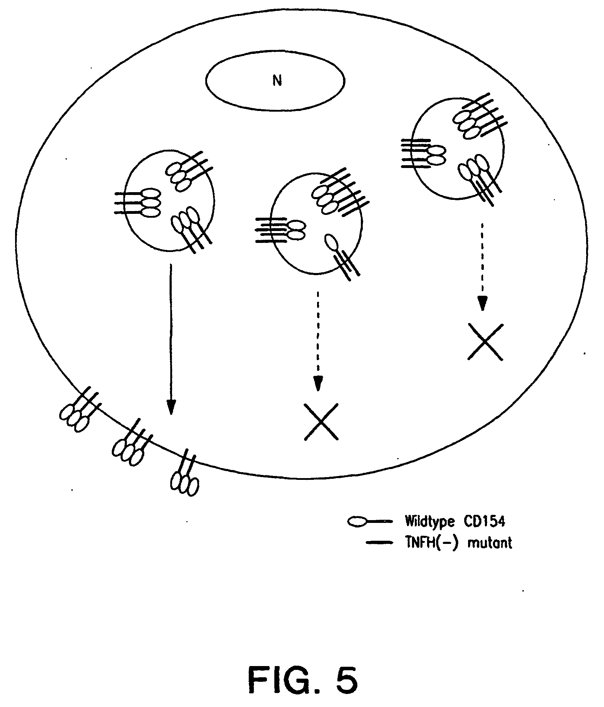

Fig. 5 illustrates this inhibitory process. When the wildtype and mutant proteins are co-expressed, wildtype proteins can form trimers with or without the TNFH(-) mutant associated. However, those bound by the mutant protein cannot mature onto the cell surface. The association with TNFH(-) per se does not compromise the ability of the wildtype protein to interact with the receptor, CD40.

### Example 6: Discussion of experimental results.

To study the potential dominant negative effect of the TNFH(-) variant on wildtype CD154, we co-expressed these two proteins in COS cells and examined their biochemical and functional properties. Our results indicate that by complexing with the wildtype protein, the mutant protein prevents the cell surface expression of wildtype CD154. This observation is significant in at least two aspects. First, it implies that while a patient's cells are capable of making both wildtype and TNFH(-) mutant CD154 proteins, they fail to produce functional CD154 on the cell surface and lead to a hyper-IgM phenotype. Second, our observation reveals that in addition to the TNFH domain, other parts of CD154 also participate in the assembly of CD154 trimers.

As CD154 is transiently expressed on the cell surface of T lymphocytes upon activation, even a partial reduction of surface expression of this protein will be beneficial in suppressing undesired T cell mediated immunological responses. Thus, the introduction of a CD154 variant lacking a functional TNFH domain into target T cells will provide a therapeutic opportunity by effectively blocking the expression of functional CD154 on the surface of the target cells.

## Claims

1. An in vitro method of decreasing the expression of wildtype CD154 on the surface of a cell, the method comprising the step of introducing into the cell a nucleic acid construct that directs expression of a mutant CD154 lacking a functional tumor necrosis factor homologous domain of wildtype CD154, wherein the mutant CD154 is capable of binding to wildtype CD154 inside a cell in which both mutant CD154 and wildtype CD154 are expressed, and wherein the mutant CD154 is thereby capable of rendering the wildtype CD154 unable to reach the cell surface.

2. Use of a nucleic acid construct that directs expression of a mutant CD154 lacking a functional tumor necrosis factor homologous domain of wildtype CD154, wherein the mutant CD154 is capable of binding to wildtype CD154 inside a cell in which both mutant CD154 and wildtype CD154 are expressed, and wherein the mutant CD154 is thereby capable of rendering the wildtype CD154 unable to reach the cell surface, for the preparation of a pharmaceutical composition for treating a patient suffering from or predisposed to a CD154-mediated disease, wherein the CD154-mediated disease is graft rejection, autoimmune disease or inflammatory disease.

3. Use of a nucleic acid construct that directs expression of a mutant CD154 lacking a functional tumor necrosis factor homologous domain of wildtype CD154, wherein the mutant CD154 is capable of binding to wildtype CD154 inside a cell in which both mutant CD154 and wildtype CD154 are expressed, and wherein the mutant CD154 is thereby capable of rendering the wildtype CD154 unable to reach the cell surface, for the preparation of a pharmaceutical composition for treating a patient suffering from or predisposed to a CD154-mediated disease, wherein the CD154-mediated disease is selected from the group consisting of lupus, systemic lupus erythematosis, lupus nephritis, lupus neuritis, asthma, chronic obstructive pulmonary disease, bronchitis, emphysema, multiple sclerosis, uveitis, Alzheimer's disease, traumatic brain injury, traumatic spinal cord injury, stroke, atherosclerosis, coronary restenosis, ischemic congestive heart failure, cirrhosis, hepatitis C, diabetic nephropathy, glomerulonephritis, osteoarthritis, rheumatoid arthritis, psoriasis, atopic dermatitis, systemic sclerosis, radiation-induced fibrosis, Crohn's disease, ulcerative colitis, multiple myeloma, ocular inflammatory disease and cachexia.

4. The method or use according to any one of claims 1-3, wherein said mutant CD154 lacks at least a portion of the tumor necrosis factor homologous domain of wildtype CD 154.

5. The method or use according to claim 4, wherein said mutant CD154 consists of amino acid residues 1-96 of SEQ ID NO:1.

6. The method or use according to any one of claims 1-4, wherein the nucleic acid construct comprises a virus-derived vector.

7. The method or use according to claim 6, wherein the virus-derived vector is a retroviral vector, lentiviral vector, adenoviral vector or an adeno-associated viral vector.

8. The method or use according to any one of claims 1-4, wherein the nucleic acid construct or pharmaceutical composition is introduced into the cell or the patient via viral transduction.

9. The use according to any one of claims 2-4, wherein the pharmaceutical composition is designed to be introduced into the cell or the patient in vivo or ex vivo.

10. The method or use according to any one of claims 1-4, wherein the cell is a T cell or a megakaryocyte, a mammalian cell, a human cell or a human T cell.

11. A pharmaceutical composition comprising a nucleic acid construct that directs expression of a mutant CD154 lacking a functional tumor necrosis factor homologous domain of wildtype CD154, wherein the mutant CD154 is capable of binding to wildtype CD154 inside a cell in which both mutant CD154 and wildtype CD154 are expressed, and wherein the mutant CD154 is thereby capable of rendering the wildtype CD154 unable to reach the cell surface

12. The pharmaceutical composition according to claim 11, wherein said mutant CD154 lacks at least a portion of the tumor necrosis factor homologous domain of wildtype CD154.

13. The pharmaceutical composition according to claim 12, wherein the mutant CD154 consists of amino acid residues 1-96 of SEQ ID NO: 1.

## Patentansprüche

1. In vitro-Verfahren zur Verminderung der Expression von CD154-Wildtyp auf der Oberfläche einer Zelle, wobei das Verfahren den Schritt des Einführens eines Nucleinsäurekonstruktes in die Zelle umfasst, das die Expression einer CD 154-Mutante lenkt, der eine funktionsfähige zum Tumornekrosefaktor homologe Domäne des CD154-Wildtyps fehlt, wobei die CD154-Mutante in der Lage ist, den CD154-Wildtyp im Innern einer Zelle zu binden, in der die CD154-Mutante und der CD154-Wildtyp beide exprimiert werden, und wobei die CD154-Mutante dabei den CD154-Wildtyp unfähig machen kann, an die Zelloberfläche zu gelangen.

2. Verwendung eines Nucleinsäurekonstruktes, das die Expression einer CD154-Mutante lenkt, der eine funktionsfähige zum Tumornekrosefaktor homologe Domäne des CD154-Wildtyps fehlt, wobei die CD154-Mutante in der Lage ist, den CD154-Wildtyp im Innern einer Zelle zu binden, in der die CD154-Mutante und der CD154-Wildtyp beide exprimiert werden, und wobei die CD154-Mutante dabei den CD154-Wildtyp unfähig machen kann, an die Zelloberfläche zu gelangen, für die Herstellung eines Arzneimittels zur Behandlung eines Patienten, der an einer CD154-vermittelten Krankheit leidet oder für eine CD154-vermittelte Krankheit prädisponiert ist, wobei die CD154-vermittelte Krankheit Transplantatabstoßung, eine Autoimmunkrankheit oder eine Entzündungskrankheit ist.

3. Verwendung eines Nucleinsäurekonstruktes, das die Expression einer CD154-Mutante lenkt, der eine funktionsfähige zum Tumornekrosefaktor homologe Domäne des CD 154-Wildtyps fehlt, wobei die CD 154-Mutante in der Lage ist, den CD 154-Wildtyp im Innern einer Zelle zu binden, in der die CD154-Mutante und der CD154-Wildtyp beide exprimiert werden, und wobei die CD154-Mutante dabei den CD154-Wildtyp unfähig machen kann, an die Zelloberfläche zu gelangen, für die Herstellung eines Arzneimittels zur Behandlung eines Patienten, der an einer CD154-vermittelten Krankheit leidet oder für eine CD154-vermittelte Krankheit prädisponiert ist, wobei die CD154-vermittelte Krankheit ausgewählt ist aus der Gruppe bestehend aus Lupus, systemischem Lupus erythematodes, Lupusnephritis, Lupusneuritis, Asthma, chronischobstruktiver Lungenerkrankung, Bronchitis, Emphysem, Multipler Sklerose, Uveitis, Alzheimer-Krankheit, traumatischer Gehirnverletzung, traumatischer Rückenmarksverletzung, Schlaganfall, Atherosklerose, Coronarrestenose, ischämischer kongestiver Herzinsuffizienz, Cirrhose, Hepatitis C, diabetischer Nephropathie, Glomerulonephritis, Osteoarthritis, rheumatoider Arthritis, Psoriasis, atopischer Dermatitis, systemischer Sklerose, strahleninduzierter Fibrose, Morbus Crohn, eiternder Colitis, multiplem Myelom, Augenentzündung und Kachexie.

4. Verfahren oder Verwendung nach einem der Ansprüche 1 bis 3, wobei der CD154-Mutante mindestens ein Teil der zum Tumornekrosefaktor homologen Domäne des CD154-Wildtyps fehlt.

5. Verfahren oder Verwendung nach Anspruch 4, wobei die CD154-Mutante aus den Aminosäureresten 1 bis 96 von SEQ ID NO: 1 besteht.

6. Verfahren oder Verwendung nach einem der Ansprüche 1 bis 4, wobei das Nucleinsäurekonstrukt einen Virus-abgeleiteten Vektor umfasst.

7. Verfahren oder Verwendung nach Anspruch 6, wobei der Virus-abgeleitete Vektor ein retroviraler Vektor, lentiviraler Vektor, adenoviraler Vektor oder adeno-assoziierter viraler Vektor ist.

8. Verfahren oder Verwendung nach einem der Ansprüche 1 bis 4, wobei das Nucleinsäurekonstrukt oder das Arzneimittel in die Zelle oder den Patienten über virale Transduktion eingeführt wird.

9. Verwendung nach einem der Ansprüche 2 bis 4, wobei das Arzneimittel so gestaltet ist, um in vivo oder ex vivo in die Zelle oder in den Patienten eingeführt zu werden.

10. Verfahren oder Verwendung nach einem der Ansprüche 1 bis 4, wobei die Zelle eine T-Zelle oder ein Megakaryocyt, eine Säugerzelle, eine menschliche Zelle oder eine menschliche T-Zelle ist.

11. Arzneimittel, umfassend ein Nucleinsäurekonstrukt, das die Expression einer CD154-Mutante lenkt, der eine funktionsfähige zum Tumornekrosefaktor homologe Domäne des CD154-Wildtyps fehlt, wobei die CD154-Mutante in der Lage ist, den CD154-Wildtyp im Innern einer Zelle zu binden, in der die CD154-Mutante und der CD154-Wildtyp beide exprimiert werden, und wobei die CD154-Mutante dabei den CD154-Wildtyp unfähig machen kann, an die Zelloberfläche zu gelangen.

12. Arzneimittel nach Anspruch 11, wobei der CD154-Mutante mindestens ein Teil der zum Tumornekrosefaktor homologen Domäne des CD154-Wildtyps fehlt.

13. Arzneimittel nach Anspruch 12, wobei die CD154-Mutante aus den Aminosäureresten 1 bis 96 von SEQ ID NO: 1 besteht.

## Revendications

1. Méthode in vitro pour diminuer l'expression de CD154 du type sauvage à la surface d'une cellule, la méthode comprenant l'étape d'introduire, dans la cellule, une construction d'acide nucléique qui dirige l'expression d'un CD154 mutant manquant d'un domaine fonctionnel homologue au facteur de nécrose de la tumeur de CD154 du type sauvage, où CD154 mutant est capable de liaison à CD154 du type sauvage à l'intérieur d'une cellule dans laquelle sont exprimés CD154 mutant et CD154 du type sauvage, où CD154 mutant est ainsi capable de rendre CD154 du type sauvage incapable d'atteindre la surface de la cellule.

2. Utilisation d'une construction d'acide nucléique qui dirige l'expression d'un CD154 mutant manquant d'un domaine homologue fonctionnel du facteur de nécrose de la tumeur de CD154 du type sauvage, où CD154 mutant est capable de liaison à CD154 du type sauvage à l'intérieur d'une cellule dans laquelle CD154 mutant et CD154 du type sauvage sont exprimés et où CD154 mutant est ainsi capable de rendre CD154 du type sauvage incapable d'atteindre la surface de la cellule, pour la préparation d'une composition pharmaceutique pour le traitement d'un patient souffrant de ou prédisposé à une maladie provoquée par CD154, où la maladie provoquée par CD154 est un rejet de greffe, une maladie auto-immune ou une maladie inflammatoire.

3. Utilisation d'une construction d'acide nucléique qui dirige l'expression d'un CD154 mutant manquant d'un domaine homologue fonctionnel du facteur de nécrose de la tumeur de CD154 du type sauvage, où CD154 mutant est capable de liaison à CD154 du type sauvage à l'intérieur d'une cellule dans laquelle CD154 mutant et CD154 du type sauvage sont exprimés et où CD154 mutant est ainsi capable de rendre CD154 du type sauvage incapable d'atteindre la surface de la cellule, pour la préparation d'une composition pharmaceutique pour le traitement d'un patient souffrant de ou prédisposé à une maladie provoquée par CD154, où la maladie provoquée par CD154 est sélectionnée dans le groupe consistant en lupus, lupus erythematosis généralisé, lupus nephritis, lupus neuritis, asthme, maladie pulmonaire obstructive chronique, bronchite, emphysème, sclérose en plaques, uvéite, maladie de Alzheimer, lésion traumatique du cerveau, lésion traumatique de la moëlle épinière, attaque, athérosclérose, resténose coronaire, asystolie congestive, cirrhose, hépatite C, néphropathie diabétique, glomérulonéphrite, ostéoarthrite, arthrite rhumatoïde, psoriasis, dermatite atopique, sclérose généralisée, fibrose induite par les rayons, maladie de Crohn, colite ulcérative, myélome multiple, maladie oculaire inflammatoire et cachexie.

4. Méthode ou utilisation selon l'une quelconque des revendications 1-3, où ledit CD154 mutant manque d'une portion du domaine homologue du facteur de nécrose de la tumeur de CD154 du type sauvage.

5. Méthode ou utilisation selon la revendication 4, où ledit CD154 mutant consiste en résidus d'acides aminés 1-96 de SEQ ID NO:1.

6. Méthode ou utilisation selon l'une quelconque des revendications 1-4, où la construction d'acide nucléique comprend un vecteur dérivé d'un virus.

7. Méthode ou utilisation selon la revendication 6, où le vecteur dérivé d'un virus est un vecteur rétroviral, un vecteur lentiviral, un vecteur adénorival ou un vecteur viral adéno-associé.

8. Méthode ou utilisation selon l'une quelconque des revendications 1-4, où la construction d'acide nucléique ou composition pharmaceutique est introduite dans la cellule ou le patient via transduction virale.

9. Utilisation selon l'une quelconque des revendications 2-4, où la composition pharmaceutique est conçue pour être introduite dans la cellule ou le patient in vivo ou ex vivo.

10. Méthode ou utilisation selon l'une quelconque des revendications 1-4, où la cellule est une cellule T ou un mégacaryocyte, une cellule mammalienne, une cellule humaine ou une cellule T humaine.

11. Composition pharmaceutique comprenant une construction d'acide nucléique qui dirige l'expression d'un CD154 mutant manquant d'un domaine fonctionnel homologue au facteur de nécrose de la tumeur de CD154 du type sauvage, où CD154 mutant est capable de liaison à CD154 du type sauvage à l'intérieur d'une cellule dans laquelle CD154 mutant et CD154 du type sauvage sont exprimés, et où CD154 mutant est ainsi capable de rendre CD154 du type sauvage incapable d'atteindre la surface de la cellule.

12. Composition pharmaceutique selon la revendication 11, où ledit CD154 mutant manque au moins d'une portion du domaine homologue au facteur de nécrose de la tumeur de CD154 du type sauvage.

13. Composition pharmaceutique selon la revendication 12, où CD154 mutant consiste en résidus d'acides aminés 1-96 de SEQ ID NO:1.


### SEQUENCE LISTING

<110> BIOGEN, INC.
<120> CD154 VARIANT.
<130> A103PCT (00455.191)
<140>
   <141>
<160> 1
<170> PatentIn Ver. 2.1
<210> 1
   <211> 261
   <212> PRT
   <213> Homo sapiens
<400> 1